# EUROPÄISCHE PATENTSCHRIFT

(11) **EP 3 030 243 B1**
(45) Veröffentlichungstag und Bekanntmachung des Hinweises auf die Patenterteilung: **26.07.2017**
(21) Anmeldenummer: 14748179.0
(22) Anmeldetag: 01.08.2014
(51) Int. Cl.: C07D 495/04, G01N 33/82, C07C 401/00

(54) **25-OH VITAMIN D DERIVATE ZUR BESTIMMUNG VON VITAMIN D METABOLITEN**
25-OH VITAMIN D DERIVATIVES FOR DETERMINATION OF VITAMIN D METABOLITES
DÉRIVÉS 25-OH DE VITAMINE D POUR DÉTERMINER DES MÉTABOLITES DE VITAMINE D

(30) Priorität: 07.08.2013 DE 102013215580
(43) Veröffentlichungstag der Anmeldung: 15.06.2016
(73) Patentinhaber: Orgentec Diagnostika GmbH, 55129 Mainz (DE)
(72) Erfinder: SOSKIC, Vukic, 55131 Mainz (DE); POPPE, Robert, 55128 Mainz (DE)
(74) Vertreter: Weiss, Wolfgang
(86) Internationale Anmeldenummer: PCT/EP2014/066620
(87) Internationale Veröffentlichungsnummer: WO 2015/018757

(56) Entgegenhaltungen:
- WO-A1-97/24127
- US-A- 4 119 647

## Beschreibung

Die vorliegende Erfindung betrifft neue Vitamin D-Verbindungen, die am C-3 Stereozentrum über einen Linker mit einer Markierungsgruppe verbunden sind. Die vorliegende Erfindung betrifft weiterhin ein Verfahren zur Herstellung dieser Vitamin D-Verbindungen sowie die Verwendung eines Zwischenprodukts zur Herstellung dieser Verbindungen.

Ein weiterer Gegenstand der vorliegenden Erfindung ist ein Verfahren zur quantitativen Bestimmung von Vitamin D unter Verwendung einer erfindungsgemäßen Vitamin D-Verbindung als Tracer. Weiterhin betrifft die vorliegende Erfindung ein Reagenz zur Bestimmung von Vitamin D, enthaltend eine erfindungsgemäße Verbindung, sowie dessen Verwendung zur Bestimmung von Vitamin D.

Für den Menschen ist eine ausreichende Versorgung mit Vitamin D essenziell. Die häufigsten physiologischen Formen von Vitamin D sind Vitamin D₃ oder Vitamin D₂. Die wichtigsten physiologisch wirksamen Derivate von Vitamin D₃ (Cholecalciferol) sind 250HD3 (25-Hydroxyvitamin D₃ oder Calcidiol) und 1,25-(OH)₂ D₃ (1-alpha, 25-Dihydroxyitamin D₃ oder Calcitriol). In der Regel wird die überwiegende Menge Vitamin D₃ nicht wie andere Vitamine mit der Nahrung aufgenommen, sondern unter dem Einfluss von UV-Licht in der Haut hergestellt. Hierbei wird 7-Dihydroxycholesterol durch UV-Bestrahlung zu Provitamin D₃ umgewandelt, wobei sich das instabile Zwischenprodukt zu Vitamin D₃ umstrukturiert. In der Nahrung ist Vitamin D₃ besonders in fettreichem Fisch enthalten. Eine weitere Form von Vitamin D, die im Körper vorkommen kann, ist Vitamin D₂ (Calciferol oder Ergocalciferol), welches in Pilzen vorkommt und mit der Nahrung aufgenommen werden kann. Vitamin D₃- und Vitamin D₂-Präparate stellen eine weitere Quelle zur Versorgung mit einer ausreichenden Menge an Vitamin D dar.

Vitamin D und seine natürlichen Derivate sind äußerst hydrophobe Moleküle. Im Blut erfolgt der Transport von Vitamin D im Komplex mit Vitamin D-Binde-Protein (DBP). DBP gehört zur Familie der Albumine und bindet Vitamin D, Vitamin D Metaboliten und Fettsäuren.

In der Leber wird Vitamin D durch Cytochrom P450 an Position C-25 oxidiert. Das resultierende 250HD (25-Hydroxyvitamin D₃ oder 25-Hydroxyvitamin D₂) ist der hauptsächlich in der Zirkulation vorliegende Vitamin D Metabolit. Die Konzentration von 250HD in Serum dient als Indikator zur Bewertung des Vitamin D-Status des Menschen.

In der Niere und in weiteren Geweben erfolgt die Umsetzung von 250HD zu 1,25(OH)₂D, der physiologisch aktiven Form des Vitamin D, welche nach Bindung an den Vitamin D Rezeptor (VDR) Vitamin D-abhängig regulierte (VDRE) Genaktivitäten steuert.

Eine ausreichende Versorgung mit Vitamin D ist für den normalen Knochenaufbau und die Regulation der Calcium- und Phosphatresorption wesentlich. Die Aktivierung des VDR mit 1,25(OH)₂D steigert die Effizienz der intestinalen Calciumaufnahme um ca. 30 % und die Phosphataufnahme um ca. 80 %. Wenn die Serumkonzentration von 250HD unter 30 ng/ml absinkt, ist dies mit einer deutlichen Verringerung der Calciumresorption im Darm und einem Anstieg der Parathyroidhormonkonzentration verbunden. Vitamin D-Mangel gilt als wesentlicher Faktor für Osteoporose mit erhöhtem Knochenbruchrisiko und ist mit weiteren Gesundheitsrisiken verbunden. Während die Vitamin D-Konzentration im Serum abhängig von der Nahrungsaufnahme und der Sonnenexposition starken Schwankungen unterliegt, gilt die 250HD-Serumkonzentration als ein guter Indikator zur Ermittlung des Vitamin D-Versorgungsstatus. Die Bestimmung der 250HD-Konzentration in Serum erfolgt daher in der medizinischen Diagnostik routinemäßig.

Die 250HD-Bestimmung in Serum erfolgt vorzugsweise durch kompetitive Bindungsanalyse. Hierbei wird die 250HD Konzentration der Probe über die Verdrängung eines radioaktiv oder chemisch markierten Vitamin D Derivates von einem Vitamin D Bindeprotein, z. B. einem 250HD-spezifischen Antikörper bestimmt. Das in dem kompetitiven Bindungsassay als Tracer verwendete markierte Vitamin D Derivat muss dabei mit hoher Affinität und Spezifität an das im Assay verwendeten Vitamin D Bindeprotein binden.

DE 69713138 (Immundiagnostic Systems Ltd.) beschreibt an der C20-Position radioaktiv modifizierte 1,25-Dihydroxyvitamin D Derivate, die als Tracer in Immunoassays verwendet werden können.

US 5,116,573 (Kureha Kagaku Kogyo Kabushiki Kaisha) beschreibt an der C19- und C6-Position Deuterium- oder Tritium-markierte Vitamin D Derivate. WO 97/24127 offenbart markierte Vitamin D Verbindungen und deren Anwendung. Alternativ kann die 3β-Hydroxygruppe von Vitamin D3 und Vitaminderivaten zur Einführung nichtradioaktiver Markierungsgruppen verwendet werden, wobei die Hydroxygruppe zu einer Ester- oder Etherbrücke modifiziert wird. Die Nutzbarkeit von Vitamin D Tracern auf der Basis von Hapten markierten Esterderivaten wird jedoch beispielsweise durch die Instabilität der Esterbrücke bei pH-Werten oberhalb von pH 7 und deren Anfälligkeit gegenüber Esterasen, die in biologischen Flüssigkeiten und Geweben häufig vorkommen, stark eingeschränkt. Um dieses Problem zu lösen, wurden Verfahren zur Umwandlung der Hydroxygruppe in ein 3β-Aminopropyletherderivat entwickelt (Roy, A. und Ray, R., Steroids. 1995, 60: 530-533, US6291693: Holick, M.F. and Ray, US6787660: Armbruster, M.P. et al.*,* US20080317764: Huber, E. et al.*,* US8003400: Kobold, U.)

Diese im Stand der Technik beschriebenen Verfahren zur Herstellung von Vitamin D Derivaten und deren Nutzung als Tracer in einem kompetitiven Bindungsassay sind jedoch mit einer Reihe von Nachteilen behaftet. Radioaktiv markierte Vitamin D Tracer weisen eine geringere Haltbarkeit und verminderte Stabilität auf. Neben den grundsätzlichen Nachteilen für die Gesundheit und die Umwelt erhöhen sich bei radioaktiven Reagenzien die Kosten für Lagerung, Transport und Entsorgung.

Die an der 3β-Hydroxygruppe modifizierten Vitamin D Derivate weisen den Nachteil auf, dass der Syntheseweg äußerst arbeitsaufwändig ist und hochentflammbare und aggressive Reagenzien unter strikt anaeroben Bedingungen eingesetzt werden müssen. Hinzu kommt, dass die Verbindungen unter physiologischen Bedingungen zum Teil nur eine geringe Stabilität aufweisen, was ihren Einsatz in Proben biologischen Ursprungs stark einschränkt.

Ein zur Bestimmung von Vitamin D verwendeter Tracer sollte synthetisch einfach zugänglich sein, keine potenziell gesundheitsschädlichen Markierungsgruppen zur Detektion enthalten und für den Einsatz in automatisierbaren Testverfahren geeignet sein.

Eine Aufgabe der vorliegenden Erfindung ist es daher, eine markierte Vitamin D-Verbindung bereitzustellen, welche die bisherigen Probleme bei der Synthese von markierten Vitamin D Derivaten löst und für eine Verwendung als Tracer in einem automatisierten Verfahren zur quantitativen Bestimmung von Vitamin D geeignet ist.

Die Einschränkungen und Nachteile der im Stand der Technik beschriebenen markierten Vitamin D-Verbindungen sowie ihrer Herstellung werden durch die vorliegende Erfindung zumindest teilweise behoben.

Die vorliegende Erfindung stellt markierte Vitamin D-Verbindungen zur Verfügung, die sich als Tracer in Testverfahren zur Bestimmung von Vitamin D eignen und über einen einfachen und kurzen Zugang synthetisch verfügbar sind.

Überraschenderweise wurde gefunden, dass eine Markierungsgruppe in den Vitamin D-Grundkörper durch nukleophile Substitution der Hydroxygruppe am C-3 Atom eingebracht werden kann. Hierbei kommt es zu einer Inversion der Konfiguration am C-3 Atom. Dennoch eignen sich die resultierenden Verbindungen mit einer Markierungsgruppe in α-Konfiguration am C-3 Atom überraschenderweise hervorragend als Tracer in kompetitiven Vitamin D-Bestimmungsverfahren. Vorteilhaft ist die inverse Konformation an C3 vor allem aufgrund einer damit einhergehenden Erhöhung der Stabilität in biologischen Flüssigkeiten wie Blut, beispielsweise gegenüber Esterasen.

Durch die vorliegende Erfindung werden neue Tracer sowie ein zuverlässiges und automatisierbares Verfahren zur Bestimmung der Konzentration von Vitamin D, insbesondere von 25-Hydroxyvitamin D₃ oder 25-Hydroxyvitamin D₂, bereitgestellt.

Ein erster Aspekt der vorliegenden Erfindung betrifft daher eine Vitamin D-Verbindung mit einer Struktur nach Formel (I), 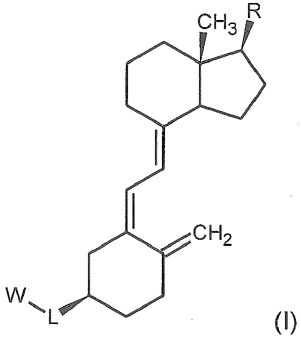 wobei
R ein C₂ - C₁₀ Alkyl- oder Alkenylrest ist, der gegebenenfalls durch eine Hydroxygruppe substituiert ist,
L ein Linker ist, und
W eine Markierungsgruppe ist, die keine Vitamin D-Verbindung ist.

Der Ausdruck "*C₂* - *C₁₀ Alkyl*" bezeichnet lineare oder verzweigte Alkylgruppen, welche 2 bis 10 Kohlenstoffatome aufweisen. Dieser Begriff umfasst Gruppen wie Ethyl, n-Propyl, iso-Propyl, n-Butyl, iso-Butyl, tert-Butyl, n-Pentyl, n-Hexyl, n-Heptyl, n-Octyl, 2-Ethylhexyl, 2-Octyl, 3-Octyl, iso-Octyl, n-Nonyl, 2-Nonyl, 3-Nonyl, 4-Nonyl, iso-Nonyl, n-Decyl, iso-Decyl, 2-Decyl und 3-Decyl. Jedes Wasserstoffatom kann auch durch ein Deuteriumatom ersetzt sein.

Der Ausdruck *"C₂* - *C₁₀ Alkenyl"* bezeichnet Alkenylgruppen, welche 2 bis 10 Kohlenstoffatome aufweisen. Diese können geradkettig oder verzweigt sein und in der Z- oder E-Form vorliegen. Solche Gruppen beeinhalten Vinyl, Propenyl, 1-Butenyl, iso-Butenyl, 2-Butenyl, 1-Pentenyl, (Z)-2-Pentenyl, (E)-2-Pentenyl, (Z)-4-Methyl-2-Pentenyl, (E)-4-Methyl-2-Pentenyl, Pentadienyl, z. B. 1,3- oder 2,4-Pentadienyl, (E)-5,6 Dimethylhept-3-enyl und (Z)-5,6 Dimethylhept-3-enyl. Jedes Wasserstoffatom kann auch durch ein Deuteriumatom ersetzt sein.

Der Ausdruck *"Markierungsgruppe"* bezeichnet eine Gruppe, welche durch physikalische, chemische und/oder biochemische Verfahren nachweisbar, mit der Verwendung des markierten Moleküls kompatibel und insbesondere nicht radioaktiv ist. Dabei handelt es sich nicht um eine Vitamin D-Verbindung mit einer Struktur nach Formel (I).

Der Ausdruck *"Linker"* bezeichnet einen molekularen Abstandshalter, der ein Vitamin D-Grundgerüst nach Formel (I) mit einer Markierungsgruppe verbindet.

In einer Ausführungsform ist der Linker dadurch gekennzeichnet, dass er eine Kettenlänge von 6 bis 110 Atomen, ausgewählt aus C-Atomen, und gegebenenfalls Heteroatomen wie etwa N, O und/oder S aufweist. Die Kettenlänge ist bevorzugt 8-80, 12-60 oder 30-50 Atome lang. Die gegebenenfalls im Linker enthaltenen Heteroatome sind bevorzugt N und/oder O.

In einer Ausführungsform ist der Linker durch die Formel (II) dargestellt: 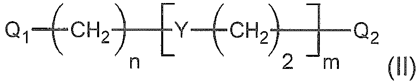 wobei
Q2: -C(O)NR¹-●, -C(O)O-●, -C(S)NR¹-●, -C(S)O-●, -C(O)S-●, -C(S)S-●, -O-●, -S-●, -NR¹-●, -CH₂O-●, -CH₂S-● oder -CH₂NR¹-● ist,
Q1: -C(O)-●, -NR²C(O)-●, -NR²C(S)-●, -OC(O)-●, -C(O)NR²-●, -C(S)NR²-●, -C(S)-●, -OC(S)-●, -SC(O)-●, -SC(S)-●, -O-●, -S-● oder -NR²-● ist,
Y: -O- oder -CH₂- ist,
   R1 und R2 unabhängig voneinander H oder C₁- C₆Alkyl sind, und
m1 - 30 ist, und
n1 - 12 ist,
wobei Q1 mit der Gruppe W und Q2 mit dem Grundkörper der Vitamin D-Verbindung nach Formel (I) verbunden ist und (●) jeweils die Verknüpfungsstelle von Q2 mit dem Grundkörper der Vitamin D-Verbindung bzw. von Q1 mit der Gruppe W angibt. Jedes Wasserstoffatom kann auch durch ein Deuteriumatom ersetzt sein.

Der Ausdruck "*C₁* - *C₆ Alkyl"* bezeichnet lineare oder verzweigte Alkylgruppen, welche 1 bis 6 Kohlenstoffatome, insbesondere 1-3 Kohlenstoffatome aufweisen. Diese Gruppen beinhalten Methyl, Ethyl, n-Propyl, iso-Propyl, n-Butyl, iso-Butyl, tert-Butyl, n-Pentyl, Neopentyl, 1-Ethylpropyl, n-Hexyl und iso-Hexyl. Jedes Wasserstoffatom kann auch durch ein Deuteriumatom ersetzt sein.

Bevorzugte Ausführungsformen sind dadurch gekennzeichnet, dass
Q2: -C(O)NR¹-●, -C(O)O-●, -C(S)NR¹-●, -C(S)O-●, -C(O)S-●, -C(S)S-● ist,
Q1: -C(O)-●, -NR²C(O)-●, -NR²C(S)-●, -OC(O)-●, -C(O)NR²-●, -C(S)NR²-●, -C(S)-●, -OC(S)-●, -SC(O)-●, -SC(S)-●, -O-●, -S-● oder-NR²-● ist,
Y: -O- oder -CH₂- ist,
R¹ und R² unabhängig voneinander H oder C₁- C₆ Alkyl sind, und m 1 - 30 ist, und
n 1 - 12 ist,
oder
Q2: -C(O)NR¹-●, -O-●, -S-●, -NR¹-●, -CH₂O-●, -CH₂S-● oder -CH₂NR¹-● ist,
Q1: -C(O)-●, -NR²C(O)-●, -NR²C(S)-●, -OC(O)-●, -C(O)NR²-, -C(S)NR²-●, -C(S)-●, -OC(S)-●, -SC(O)-●, -SC(S)-●, -O-●, -S-● oder -NR²-● ist,
Y: -O- oder -CH₂- ist,
R¹ und R² unabhängig voneinander H oder C₁ - C₆ Alkyl sind, und
m 1 - 30 ist, und
n 1 - 12 ist.

Eine weitere bevorzugte Ausführungsform ist dadurch gekennzeichnet, dass
Q2: -C(O)NR¹-● ist,
Q1: -C(O)-●, -NR²C(O)-●, -OC(O)-●, -C(O)NR²-●, -O-●, -S-● oder -NR²-● ist,
Y: -O- ist,
R¹ und R² unabhängig voneinander H oder C₁ - C₆ Alkyl und insbesondere H sind,
m 1 - 30 ist, und
n 1 -12 ist.

Die Gruppe Y in einem Linker nach Formel (II) ist bevorzugt -O-.

In einer anderen bevorzugten Ausführungsform weist die Vitamin D-Verbindung eine Struktur nach Formel (la) auf 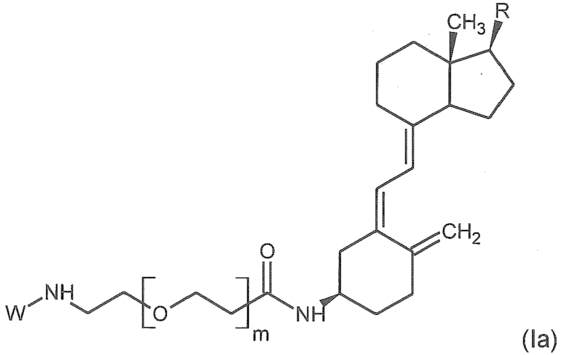 wobei
R ein C₂ - C₁₀ Alkyl- oder Alkenylrest ist, der gegebenenfalls durch eine Hydroxygruppe substituiert ist,
m 1-30 ist, und
W eine Markierungsgruppe ist, die keine Vitamin D-Verbindung ist.

In noch einer anderen bevorzugten Ausführungsform hat die Vitamin D-Verbindung eine Struktur nach Formel (Ib) 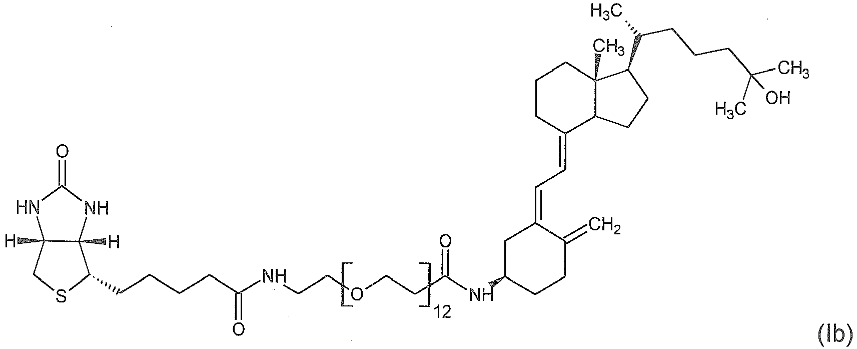 oder nach Formel (Ic)

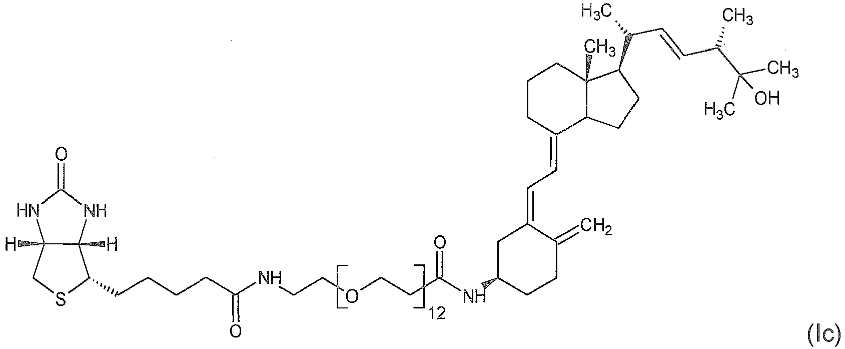

Die Markierungsgruppe W kann einerseits eine Gruppe sein, die direkt nachweisbar ist, oder andererseits eine Gruppe, die indirekt nachweisbar ist, z. B. nach Bindung mit einem komplementären, nachweisbaren Bindepartner.

Direkt nachweisbare Gruppen sind beispielsweise Farbstoffe, Enzyme, elektrochemisch nachweisbare Gruppen oder Lumineszenzgruppen wie Fluoreszenzgruppen, Phosphoreszenzgruppen oder Chemolumineszenzgruppen, die z. B. mit optischen Verfahren nachgewiesen werden können.

Unter einer elektrochemisch nachweisbaren Gruppe ist eine Gruppe zu verstehen, die mit einem Reaktionspartner eine Elektronenübertragungsreaktion eingehen kann, wobei Elektronen von dem einen Reaktionspartner auf den anderen übertragen werden. Die nachweisbare Gruppe wird entweder reduziert und der Reaktionspartner oxidiert, oder die nachweisbare Gruppe wird oxidiert und der Reaktionspartner reduziert.

Unter einer Fluoreszenzgruppe ist ein fluoreszierender Farbstoff zu verstehen, der nach Anregung spontan Licht emittiert, das energieärmer als das vorher absorbierte ist. Bevorzugt handelt es sich bei der Fluoreszenzgruppe um Farbstoffe wie beispielsweise Fluorescein, Rhodamin, Stilben, fluoreszierende Proteine wie Green Fluorescent Protein (GFP), Yellow Fluorescent Protein (YFP) und Red Fluorescent Protein (RFP), Cumarin, Chinin, Allophycocyanin, synthetische Fluoreszenzlabel beziehungsweise -marker wie z. B. ATTO-dyes (ATTO-TEC GmbH, Siegen), Alexa-Fluor (Molecular Probes, Invitrogen Corp., Carlsbad) und Cyanine wie Cy3, Cy5 und Cy7 sowie Derivate davon.

Unter einer Phosphoreszenzgruppe ist eine chemische Gruppe zu verstehen, die nach Anregung über einen längeren Zeitraum Licht emittiert, wodurch es zu einem Nachleuchten von Sekundenbruchteilen bis Stunden kommt.

Bei einer Chemolumineszenzgruppe handelt es sich um eine chemische Gruppe, die durch eine chemische Reaktion elektromagnetische Strahlung im Bereich des sichtbaren Lichts oder im Infrarotbereich emittiert. Chemolumineszierende Markierungen umfassen Luminol, Acridiniumester und Polyhistidin-Markierungen.

Indirekt nachweisbare Gruppen sind beispielsweise Biotin, das durch markiertes Avidin oder Steptavidin nachgewiesen werden kann, Antigene und Haptene, die durch markierte, spezifische Antikörper nachgewiesen werden können, Kohlenhydratstrukturen, die durch Lektine nachgewiesen werden können, oder Affinitäts-Tags.

Der Ausdruck Biotinyl umfasst auch Derivate von Biotinyl, sofern diese an Streptavidin oder Avidin binden.

In einer bevorzugten Ausführungsform ist W Biotinyl, eine Fluoreszenzgruppe oder eine Chemolumineszenzgruppe.

In einer anderen bevorzugten Ausführungsform ist W Biotinyl.

Die Gruppe R ist bevorzugt eine Vitamin D-Seitenkette, insbesondere mit einer OH-Gruppe, wie etwa eine Struktur nach Formel (III): 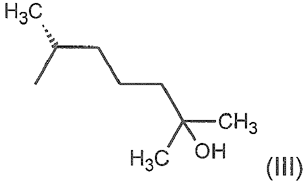 oder eine Struktur nach Formel (IV)

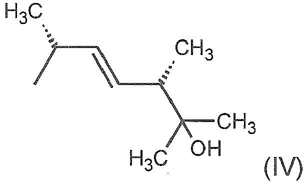

Besonders bevorzugt weist R eine Struktur nach Formel (III) auf.

Ein zweiter Aspekt umfasst ein Verfahren zur Herstellung einer Vitamin D-Verbindung mit einer Struktur nach Formel (I), umfassend die Schritte:
(a) Durchführen einer nukleophilen Substitution an der Hydroxygruppe des C-3 Atoms einer Verbindung mit einer Struktur nach Formel (V): 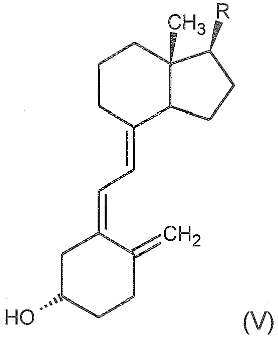 wobei R ein C₂ - C₁₀ Alkyl- oder Alkenylrest ist, der gegebenenfalls durch eine Hydroxygruppe substituiert ist, wobei die Hydroxygruppe unter Inversion der Konfiguration am C-3-Stereozentrum durch eine Gruppe X ersetzt wird, um eine Verbindung mit einer Struktur nach Formel (VI) zu erhalten, 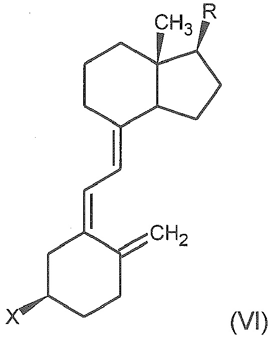 wobei R ein C₂ - C₁₀ Alkyl- oder Alkenylrest ist, der gegebenenfalls durch eine Hydroxygruppe substituiert ist, und X eine gegebenenfalls geschützte Hydroxygruppe, Thiolgruppe oder primäre oder sekundäre Aminogruppe ist, und
(b) Kuppeln einer Markierungsgruppe W, die keine Vitamin D-Verbindung ist, über einen Linker an die Gruppe X der Verbindung mit einer Struktur nach Formel (VI), um eine Verbindung mit einer Struktur nach Formel (I) zu erhalten.

Die in Schritt (a) durchgeführte nukleophile Substitution umfasst bevorzugt ein Umsetzen des Alkohols (V) mit einem Alkylphosphin, z. B. Tri-n-butylphosphin, oder einem Arylphosphin, z. B. Triphenylphosphin oder Diphenyl(2-pyridyl)phosphin, einem Alkylazodicarboxylat wie Diethylazodicarboxylat oder Diisopropylazodicarboxylat und einer nukleophilen Gruppe X, bevorzugt einer leicht sauren nukleophilen Gruppe, in einem geeigneten Lösungsmittel wie Tetrahydrofuran oder Diethylether.

Die nukleophile Gruppe X kann in geschützter oder freier Form eingeführt werden. Vorzugsweise wird die Gruppe X in einer geschützten Form eingeführt.

Eine *"geschützte"* Form bedeutet, dass es sich um eine Vorläufergruppe handelt, die beispielsweise, je nach Funktionalität, geeignete Schutzgruppen aufweist, welche dem Fachmann gut bekannt sind, wobei diese geschützte Form in die entsprechende ungeschützte Gruppe umgewandelt werden kann durch im Stand der Technik bekannte Verfahren.

Die Verbindung zur Einführung der Gruppe X wird vorzugsweise ausgewählt aus der Gruppe umfassend Imide, Amine, Stickstoffwasserstoffsäure, Carbonsäuren, Alkohole, Thiole, Thiocarbonsäuren oder Salzen davon. Die Verbindung ist bevorzugt ein Imid, Amin oder Stickstoffwasserstoffsäure, bevorzugter ein Imid oder Stickstoffwasserstoffsäure und am stärksten bevorzugt ein Phthalimid oder Salze davon. Diese Gruppen können, wenn nötig, durch entsprechende Verfahren, die dem Fachmann bekannt sind, in eine entsprechende Hydroxygruppe, Thiolgruppe oder primäre oder sekundäre Aminogruppe entschützt werden. Beispielsweise kann Phthalimid durch primäre Amine wie Methylamin oder Hydrazin in einem geeigneten Lösungsmittel in das entsprechende primäre Amin umgewandelt werden. Die Gruppe R ist bevorzugt eine Vitamin D-Seitenkette, bevorzugt mit einer OH Gruppe, wie etwa eine Struktur nach Formel (III) oder nach Formel (IV).

Die Kupplung in Schritt (b) einer wie oben definierten Markierungsgruppe W über einen wie oben definierten Linker an die Gruppe X in einer Verbindung mit einer Struktur nach Formel (VI) wird nach Verfahren durchgeführt, die dem Fachmann bekannt sind. Vorzugsweise wird ein Linker, welcher bereits mit einer Markierungsgruppe W verknüpft ist, an die Gruppe X gekuppelt. Vorzugsweise weist der Linker dabei eine mit X reaktive Gruppe, beispielsweise eine Abgangsgruppe wie etwa N-Hydroxysuccinimidyl auf, die durch die Gruppe X substitutiert werden kann, wodurch die Gruppe X mit dem Linker verknüpft wird, um eine Verbindung mit einer Struktur nach Formel (I) zu ergeben.

Ein dritter Aspekt der vorliegenden Erfindung betrifft ein In-vitro-Verfahren zur quantitativen Bestimmung von Vitamin D in einer Probe unter Verwendung einer Verbindung mit einer Struktur nach Formel (I) als Tracer.

Der Ausdruck *"Tracer"* bezeichnet eine Verbindung, welche eine Gruppe aufweist, die direkt nachweisbar ist oder indirekt nachweisbar ist, z. B. nach Bindung mit einem komplementären nachweisbaren Bindepartner. Vorzugsweise wird der Nachweis mit chemischen, physikalischen oder biochemischen Verfahren durchgeführt, bevorzugt mit physikalischen und biochemischen Verfahrenen wie etwa spektroskopischen Verfahren oder immunologischen Assays.

Wenn nicht anders angegeben, schließt der Ausdruck *"Vitamin D"* alle natürlich vorkommenden Verbindungen ein, welche das Vitamin D2-Grundgerüst oder das Vitamin D3-Grundgerüst oder das Vitamin D3-Grundgerüst gemäß den Strukturformeln (VII) oder (VIII) aufweisen.

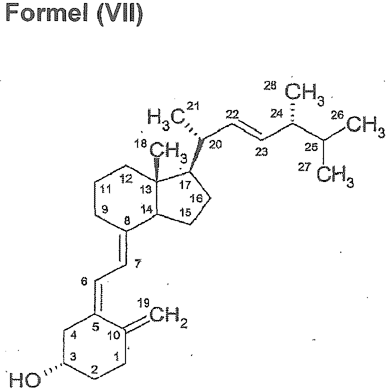

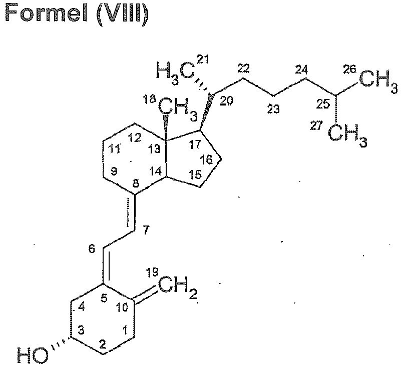

Die Nummerierung der Kohlenstoffatome in den Strukturformeln (VII) und (VIII) ist gemäß der Steroid-Nomenklatur angegeben. 25-Hydroxyvitamin D bezeichnet Vitamin D Metaboliten, welche an Position 25 der Strukturformeln (VII) oder (VIII) hydroxyliert sind, d. h. 25-Hydroxyvitamin D₂ sowie 25-Hydroxyvitamin D₃. Weitere Hydroxyvitamin D-Verbindungen sind beispielsweise 1,25-Dihydroxyvitamin D oder 24,25-Dihydroxyvitamin D.

1,25-Dihydroxyvitamin D bezieht sich auf die aktiven Formen von Vitamin D (die sogenannten D Hormone), welche an Position 1 sowie an Position 25 der Strukturformeln (VII) und (VIII) hydroxyliert sind.

Andere gut bekannte Vitamin D-Verbindungen sind 24,25-Dihydroxyvitamin D2, 24,25-Dihydroxyvitamin D3 und C3-epi-25-Hydroxyvitamin D.

In einer Ausführungsform umfasst das In-vitro-Verfahren die Schritte:
(a) In Kontakt bringen einer Vitamin D enthaltenden Probe mit dem Tracer,
(b) Quantitatives Bestimmen des Tracers unter Bedingungen, bei denen die detektierte Menge des Tracers einen Rückschluss auf die Gesamtkonzentration an Vitamin D in der Probe ermöglicht.

Das Vitamin D in Schritt (a) kann in freier Form vorliegen. In einer anderen Ausführungsform liegt das Vitamin D in gebundener Form vor, und Schritt (a) umfasst weiterhin das Freisetzen des gebundenen Vitamins D.

Der Ausdruck *"Freisetzung von gebundenem Vitamin D"* bedeutet die vollständige oder teilweise Abtrennung von Vitamin D aus Komplexen mit Proteinen, insbesondere DBP, an welche es in biologischen Proben gebunden sein kann. Vorzugsweise wird im Wesentlichen das gesamte in der Probe vorhandene Vitamin D freigesetzt. In diesem Zusammenhang bedeutet *"im Wesentlichen",* dass mindestens 90%, 95%, 98% und bevorzugt mindestens 99% des Vitamin D freigesetzt wird.

Geeignete Freisetzungsmittel sind dem Fachmann wohl bekannt und umfassen organische Substanzen wie beispielsweise Pamoasäure oder 8-Anilino-1-naphthalinsulfonsäure (8-ANS), anorganische Substanzen wie Alkalibasen in Kombination mit Reduktionsmitteln oder biochemische Substanzen wie die Serinproteinase Proteinase K.

Besonders geeignet ist ein Freisetzungsreagenz, welches mindestens eine hydrotrope Substanz wie etwa Toluolsulfonsäure, mindestens ein Übergangsmetallsalz wie etwa ein Eisen(III)Salz, beispielsweise Eisen(III)chlorid oder Eisen(III)citrat, und gegebenenfalls einen Komplexbildner wie Citrat, EDTA und/oder ein Salz davon umfasst, wie in DE 10 2013 205 055.0 beschrieben.

Die Bestimmung des Vitamin D gemäß Schritt (b) kann zeitlich nach dem Schritt (a) oder gemeinsam mit dem Schritt (a) durchgeführt werden. Vorzugsweise wird Schritt (b) gleichzeitig mit dem Schritt (a) ausgeführt, wodurch ein Ein-Schritt-Verfahren zur Bestimmung von Vitamin D in einer Probe bereitgestellt wird.

Die Bestimmung der Gesamtkonzentration des Vitamin D in Schritt (b) erfolgt bevorzugt durch Bestimmen des Tracers in einem immunologischen Verfahren, bei dem der Tracer an einen immunologischen Partner bindet, um einen Immunkomplex zu bilden. Der Ausdruck *"immunologischer Bindungspartner"* schließt Antikörper ein.

Besonders bevorzugt wird ein kompetitives immunologisches Verfahren für die Bestimmung der Konzentration von Vitamin D in Schritt (b) verwendet.

Geeignete kompetitive Testformate sind dem Fachmann gut bekannt. In einem typischen kompetitiven Bindungsassay wird ein Rezeptor oder Antikörper mit einem Tracer, d. h. einer markierten Form von Vitamin D, mit der zu untersuchenden Probe in Kontakt gebracht. Die Menge an Tracer, welcher an den Rezeptor oder Antikörper gebunden vorgefunden wird, ist dann indikativ für den Anteil an unmarkiertem Vitamin D in der Probe. Alternativ kann ein kompetitiver Bindungsassay das Inkontaktbringen eines an einen Tracer gebundenen Rezeptors oder Antikörpers mit der zu untersuchenden Probe und das Messen der Menge an verdrängtem Tracer, welche indikativ für die Menge an Vitamin D in der Probe ist, umfassen. Der Tracer weist eine wie oben definierte Markierungsgruppe auf, welche insbesondere ausgewählt wird aus Biotinyl, einer Fluoreszenzgruppe oder einer Chemoluminszenzgruppe, die wie oben definiert sind.

In der am meisten bevorzugten Ausführungsform wird ein mit Biotin markierter Tracer verwendet und eine Detektion erfolgt durch Peroxidasemarkiertes Streptavidin.

Insbesondere wird in Schritt (b) zur Bestimmung des Tracers ein Antikörper verwendet, welcher spezifisch die Seitenkette R einer Verbindung mit einer Struktur nach Formel (I) erkennt und spezifisch eine Vitamin D3- oder Vitamin D2-Seitenkette, die am C-25 Atom eine Hydroxygruppe aufweist, erkennt.

Der Tracer wird der Probe vorzugsweise in einer Menge zugesetzt, um eine Endkonzentration von 0,2-100 ng/ml, insbesondere von 5-50 ng/ml des Tracers einzustellen.

Die zu untersuchende Probe ist vorzugsweise eine biologische Flüssigkeit, die z. B. ausgewählt wird aus Blut, Serum, Plasma oder Milch. Die Probe stammt üblicherweise von einem Lebewesen, vorzugsweise von einem Säugetier, z. B. von einem Menschen.

Das durch das erfindungsgemäße Verfahren zu bestimmende Vitamin D weist am C-25 Atom eine Hydroxygruppe auf und umfasst z. B. 25-Hydroxyvitamin D3, 25-Hydroxyvitamin D2, 1,25-Dihydroxyvitamin D3, 1,25-Dihydroxyvitamin D2 und Mischungen davon. Besonders bevorzugt sind die Vitamin D-Verbindungen 25-Hydroxyvitamin D3 und/oder 25-Hydroxyvitamin D2.

Ein vierter Aspekt der vorliegenden Erfindung betrifft ein Reagenz zur Bestimmung von Vitamin D umfassend eine Verbindung nach Formel (I).

Vorzugsweise enthält das Reagenz weiterhin ein Reagenz zur Freisetzung von gebundenem Vitamin D und gegebenenfalls Verdünnungsmittel, Träger, Lösemittel, Adjuvantien, Stabilisatoren, Konservierungsmittel und/oder Dispergiermittel.

Das Reagenz liegt vorzugsweise als Trocken- oder Nassreagenz vor.

Ein fünfter Aspekt der vorliegenden Erfindung ist die Verwendung des oben beschriebenen Reagenzes zur Bestimmung von Vitamin D, insbesondere in dem erfindungsgemäßen In-vitro-Verfahren wie oben beschrieben.

Ein sechster Aspekt der vorliegenden Erfindung ist die Verwendung einer Verbindung mit einer Struktur nach Formel (VI): 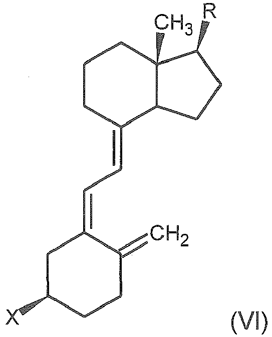
wobei R ein C₂ - C₁₀ Alkyl- oder Alkenylrest ist, der gegebenenfalls durch eine Hydroxygruppe substituiert ist,
X NHR³, OH oder SH ist, und
R³ H oder C₁-C₆ Alkyl ist,
zur Herstellung einer Tracerverbindung, d. h. einer Verbindung mit einer Struktur nach Formel (I), Formel (la) Formel (Ib) oder Formel (Ic), insbesondere zur Herstellung einer Verbindung mit einer Struktur nach Formel (I), Formel (Ib) oder Formel (Ic).

Jedes Wasserstoffatom kann auch durch ein Deuteriumatom ersetzt sein.

Die Gruppe R ist bevorzugt eine Vitamin D-Seitenkette, bevorzugt mit einer OH Gruppe, wie etwa eine Struktur nach Formel (III) oder nach Formel (IV).

Im Weiteren soll die vorliegende Erfindung detaillierter durch die folgenden Zeichnungen und Beispiele beschrieben werden.

### Figurenlegende:

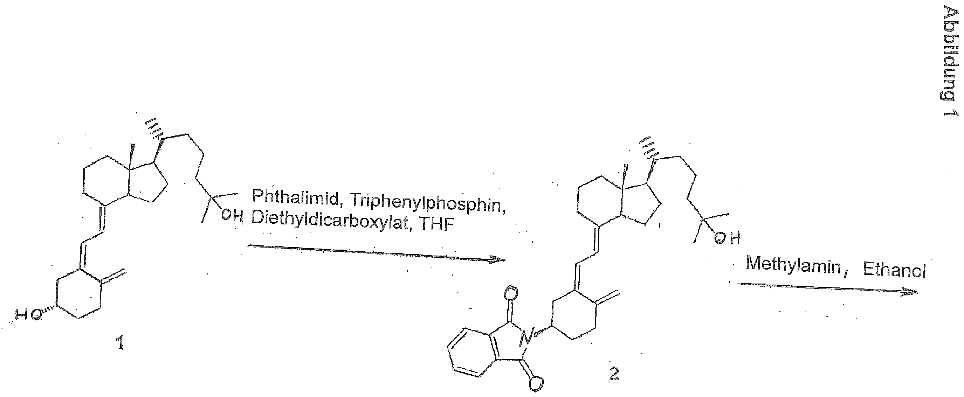

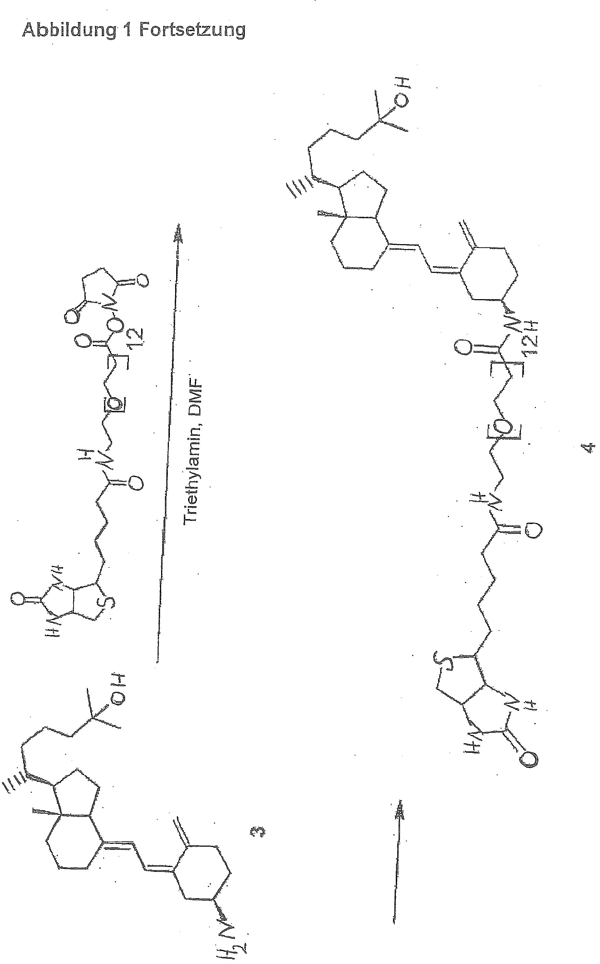

**Abb. 1****:** Synthese eines Biotin 25-OH-Vitamin D3 Tracerreagenzes durch Umwandlung von 25-Hydroxyvitamin D3 **(1)** in das epimere 3α N-Phthalimidoderivat **(2),** Entschützung zum Aminoderivat **(3)** und Umsetzung mit NHS-PEG₁₂-Biotin zum 3α-[α-Biotinamid-ω-dodeca(ethylenglycol)-amid]-25-hydroxy-deoxyvitamin D3 **(4).**

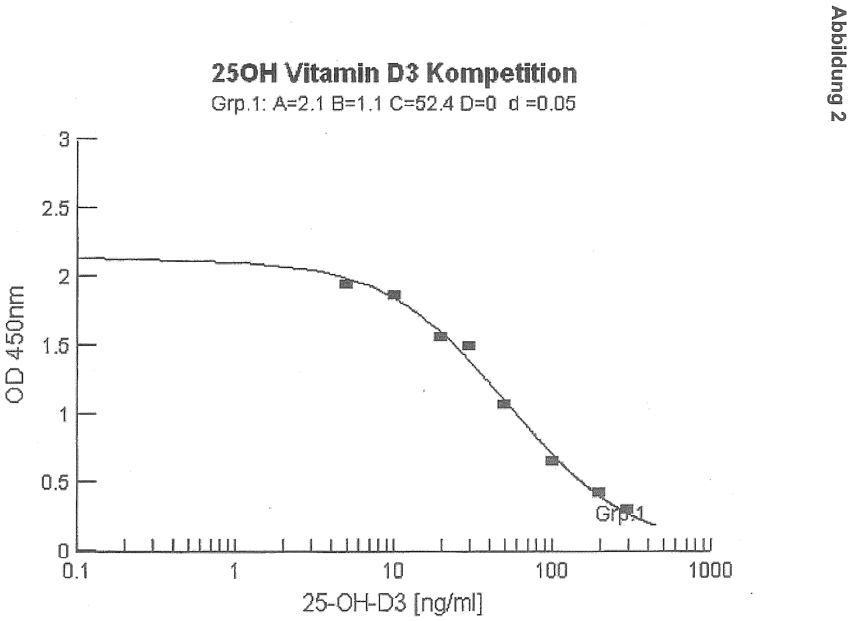

**Abb. 2****:** Charakterisierung der kompetitiven Verdrängung definierter 250HD3 Konzentrationen durch ein Biotin-25OHD3 Tracerreagenz. Zur ELISA Detektion der spezifischen 250HD Kompetition wurden Verdünnungsserien von 25OHD3 in Vitamin D-freier Serummatrix (SerCon, Seracare Inc.) in den Konzentrationen 0, 5, 10, 20, 20, 50, 100, 200, 300 ng/ml in Gegenwart von 7,5 ng/ml Biotin-25OHD Tracerreagenz mit 80 µl Vitamin D-Freisetzungsreagenz versetzt und auf Mikrotiterplattenkavitäten, die mit einem Anti 250HD Antikörper beschichtet waren, aufgetragen. Die Bindung des Tracers in Abhängigkeit von der kompetierenden 250HD Konzentration wurde mit Peroxidase-markiertem Streptavidin und einer Tetramethylbenzidin (TMB)-Farbreaktion durch Messung der OD 450 nm bestimmt.

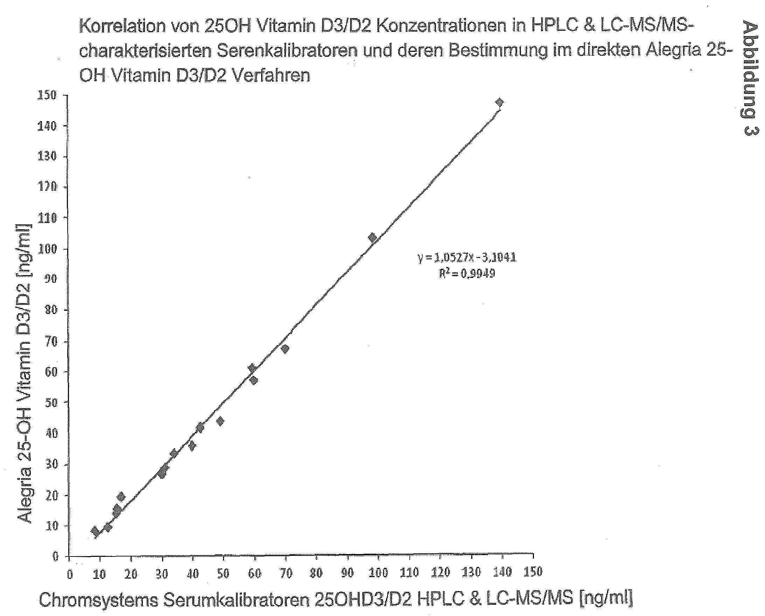

**Abb. 3****:** Die vergleichende Analyse von Seren mit definierten 250HD3 und 250HD2 Konzentrationen (6PLUS1 Multilevel Serum Calibrator Set 25-OH-Vitamin D3/D2, Chromsystems GmbH) und deren Verdünnungen in Vitamin D-freier Serummatrix (SerCon, Seracare Inc.) zeigte eine hohe Übereinstimmung zwischen den HPLC und LC-MS/MS definierten 25-OHD3/D2 Konzentrationen und dem direkten 25-OH Vitamin D3/D2 Bestimmungsverfahren im automatischen Aleria™ System (ORG270, ORGENTEC).

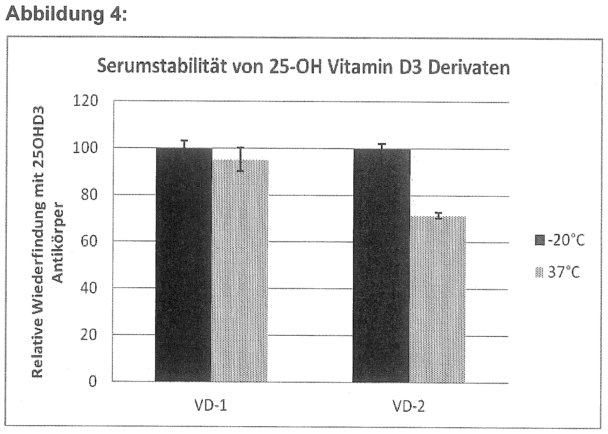

**Abb. 4****:** Die vergleichende Bestimmung der Wiederfindung von Verdünnungen der 25-OH Vitamin D Derivate 3α-[α-Biotinamido-ω-dodeca(ethylenglycol)-amido]-25-hydroxy-deoxyvitamin D₃ (VD1) und 3β-3'[α-Biotinamido-ω-dodeca(ethylenglycol)-amido]amidopropylether-25-hydroxy Vitamin D₃ (VD2) in Vitamin D-freier Serummatrix nach 24-stündiger Inkubation bei 37 °C zeigt eine hohe Serumstabilität des am C3-Atom in (R)-Konfiguration vorliegenden Derivates VD1 gegenüber dem am C3-Atom in (S)-Konfiguration vorliegenden VD2.

### Beispiel 1: Synthese von 3α-[α-Biotinamid-ω-dodeca(ethy)englycol)-amid]-25-hydroxy-deoxyvitamin D3 (Abb. 1)

¹H-NMR (bei 300 MHz) Spektren wurden auf einem Bruker AC300 (Bruker, Karlsruhe, Bruker) mit dem Lösungsmittel CDCl₃ aufgenommen. Die Auswertung erfolgte in ppm mit Tetramethylsilane als internem Standard. Die Massenspektren wurden auf einem Varian MAT 311 Spektrometer (70 eV, EI) bestimmt. Für die analytische Dünnschichtchromatographie wurden F.256 Silicagelplatten (Merck Darmstadt, Germany) verwendet. Chromatographische Reinigungen erfolgten in Silicagelsäulen, 230-400 mesh ASTM. Alle Reagenzien wurden von Sigma Aldrich bezogen.

### 1.1: Synthese von 3α-Phthalimid-25-hydroxy-deoxyvitamin D3

Eine Lösung von 25-Hydroxy-Vitamin D3 (10,0 mg, 25 µmol), Phthalimid (4,5 mg, 31,25 µmol), Triphenylphosphin (8,2 mg, 31,25 µmol) und Diethylazodicarboxylat (5,4 mg, 31,25 µmol) in Tetrahydrofuran (2,0 ml) wurde bei Raumtemperatur 24 h gerührt. Nach Zusatz von Hexan (2,0 ml) wurde die Mischung durch einen Kieselgelfilter filtriert. Nach Entfernen des Lösungsmittels wurden die Feststoffe in 5 ml 10% Na₂CO₃ aufgenommen und 2-mal mit je 10 ml Diethylether extrahiert. Die vereinigten Etherphasen wurden über MgSO₄ getrocknet und unter Vakuum zu einem farblosen Öl konzentriert. Das Produkt wurde durch Silicagelsäulenchromatographie gereinigt. 3α-Phthalimid-25-hydroxy-deoxyvitamin D3 (Abb. 1, Formel 2) wurde in Form eines farblosen Öls isoliert (7,3 mg). NMR: δ 0,58 (s, H-C(18), 3H), 0,85 (d, J=6,4 Hz, H-C(21), 3H), 1,21 (s, H-C(26 und 27), 6H), AB quartet: 4,84 (d, J=2,4Hz, H-C(19)E, 1H) und 5,06 (d, J=1,9Hz, H-C(19)Z, 1H), AB quartet: 6,02 (d, J=11,0Hz, H-C(7), 1H) und 6,26 d, J=11,0Hz, H-C(6), 1H), 7,80 (m, Ar, 4H), MS: m/e (100) 529,24 (M⁺).

### 1.2: Synthese von 3α-Amino-25-hydroxy-deoxyvitamin D3

3α-Phthalimid-25-hydroxy-deoxyvitamin D3 **(2),** (7,2 mg, 13,5 mmol) wurde in 0,5 ml 8M Methylamin in Ethanol unter Stickstoff gelöst. Die Mischung wurde zunächst 2 h bei Raumtemperatur und anschließend 16 h bei 4 °C gerührt. Das Lösungsmittel wurde unter Unterdruck abgedampft und der Rückstand wurde in 5 ml 10 % Na₂CO₃ aufgenommen und 3-mal mit je 10 ml Diethylether extrahiert. Die vereinigten Etherphasen wurden über MgSO₄ getrocknet und unter Vakuum zu einem farblosen Öl konzentriert. Das Produkt wurde durch Silicagelsäulenchromatographie gereinigt. 3α-Amino-25-hydroxy-deoxyvitamin D3 wurde als farbloses Öl isoliert **(4),** (5,2 mg). NMR: δ 0,57 (s, H-C(18), 3H), 0,85 (d, J=6,4 Hz, H-C(21), 3H), 1,21 (s, H-C(26 und 27), 6H), AB quartet: 4,83 (d, J=2,4Hz, H-C(19)E, 1H) und 5,00 (d, J=1,8Hz, H-C(19)Z, 1H), AB quartet: 5,97 (d, J=11,0Hz, H-C(7), 1H) und 6,27 d, J=11,0Hz, H-C(6), 1H), MS: m/e (100) 400,37 (M⁺).

### 1.3: Synthese von 3α-[α-Biotinamid-ω-dodeca(ethylenglycol)-amid]-25-hydroxy-deoxyvitamin D3

3α-Amino-25-hydroxy-deoxyvitamin D3 **(3),** (5,2 mg, 12,9 µmol) Triethylamin (0,02 µL), NHS-PEG₁₂-Biotin (9,4 mg, 10,0 µmol, Thermo Scientific, Prod. No. 21312) wurden in 0,2 ml Dimethylformamid gelöst und über Nacht bei Raumtemperatur gerührt. Das Lösungsmittel wurde unter Unterdruck abgedampft und der Rückstand wurde durch Silicagelsäulenchromatographie gereinigt. Hierbei wurden 5,9 mg 3α-[α-Biotinamid-ω-dodeca(ethylenglycol)-amid]-25-hydroxy-deoxyvitamin D3 **(4)** in Form eines farblosen Wachses erhalten. NMR: δ 0,58 (s, H-C(18), 3H), 0,85 (d, J=6,4 Hz, H-C(21), 3H), 1,21 (s, H-C(26 und 27), 6H), 3,62 (m, H-C(ethylenglycol), 50H), AB quartet: 4,84 (d, J=2,4Hz, H-C(19)E, 1H) und 5,06 (d, J=1,9Hz, H-C(19)Z, 1H), AB quartet: 6,02 (d, J=11,0Hz, H-C(7), 1H) und 6,26 d, J=11,0Hz, H-C(6), 1H), MS: m/e (100) 1247,80 (M+Na⁺).

### Beispiel 2: Herstellung eines Vitamin D Freisetzungsreagenzes

Natriumtoluolsulfonat wurde in Wasser gelöst und mit Stammlösungen von 1 M Natriumcitrat und 1 M Eisen(III)chlorid auf eine Endkonzentration von 1 M Natriumtoluolsulfonat, 100 mM Natriumcitrat und 50 mM Eisen(III)chlorid eingestellt.

### Beispiel 3: Bindung von einem 25-OHD Antikörper an eine feste Phase

Ein Antikörper gegen 25-OH-Vitamin D3 bzw. 25-OH-Vitamin D2 wurde in Tris-Salzpuffer pH 8,0 auf eine Konzentration von 1 µg/ml verdünnt. Die Kavitäten einer Mikrotiterplatte (Maxisorb, Nunc) wurden mit 100 µl der Antikörperverdünnung beschichtet, bei 37°C getrocknet und bis zur Testdurchführung bei 4°C gelagert.

### Beispiel 4: Kompetitive Bindungsanalyse

Vitamin D freie Serummatrix (Seracon, Seracare Inc.) wurde mit definierten Konzentrationen 25-OHD3 versetzt. Jeweils 80 µl der Konzentrationsserie wurden in eine mit 3α-[α-Biotinamid-ω-dodeca(ethyleneglycol)-amid]-25-hydroxy-deoxyvitamin D3 (250HD-Biotin-Tracerreagenz) aus Beispiel 1 als Tracer vorbelegte Mikrotiterplattenkavität gefüllt und zur Resolubilisation des Tracerreagenz 5 min bei RT (20-27 °C) inkubiert. Anschließend wurden 80 µl Freisetzungsreagenz aus Beispiel 2, welches Vitamin D der Probe aus dem Komplex mit DBP dissoziert, dem Probenansatz zugefügt und gemischt. 100 µl der mit Vitamin D-Freisetzungsreagenz verdünnten Proben wurden in 250HD-Antikörper beschichtete Kavitäten transferiert und 30 min bei RT inkubiert. Das in der Probe enthaltene 250HD konkurriert nun mit dem 25OHD-Biotin-Tracer um Bindungsstellen am 250HD Antikörper-Anschließend wird der Probenansatz entfernt, und die Kavitäten werden 3-mal mit je 200 µl Waschpuffer gespült. Die Detektion des antikörpergebundenen Biotin-25OHD Tracers erfolgte mit Peroxidase-markiertem Steptavidin und einer Tetramethylbenzidin (TMB)-Farbreaktion. Nach Abstoppen der Substratreaktion durch Zusatz von 100 µl 50 mM Phosphorsäure erfolgte die Messung der optischen Dichte bei 450 nm. Hierzu wurde wie in **Abb. 2** dargestellt bereits bei einer 250HD3 Konzentration von 5 ng/ml eine spezifische Kompetition des Biotin-25OHD Tracers nachgewiesen.

### Beispiel 5: Bestimmung von 25-Hydroxyvitamin D in Serum oder Plasma in dem automatisierten Testsytem Alegria™ 25-OH Vitamin D3/D2 Testsystem

Zur Untersuchung der Korrelation zwischen HPLC + LC-MS/MS basierten 25OHD3/D2 Bestimmungsverfahren und dem automatisierten Alegria™ 25-OH Vitamin D3/D2 Testsystem (ORG270, Orgentec Diagnostika GmbH) wurden Serum- oder Plasmaproben, deren 25OHD3/D2 Konzentration über HPLC + LC-MS/MS basierte Verfahren charakterisiert wurde (6PLUS1 Multilevel Serum Calibrator Set 25-OH-Vitamin D3/D2, Chromsystems GmbH), sowie Verdünnungsstufen hieraus bis zur Bestimmung aliquotiert bei -20°C gelagert.

Zur Bestimmung wurden je 80 µl der Proben in Kavität A des Alegria™-Teststreifens vorgelegt. Die 8 Kavitäten eines Teststreifens enthielten hierbei das Vitamin D Freisetzungsreagenz aus Beispiel 2, Streptavidin-HRP Konjugat, TMB-Substrat, eine 250HD Kalibratorlösung und 25-OHD Antikörper beschichtete Kavitäten für jeweils eine Probenbestimmung. Die Testbearbeitung erfolgte automatisch im Alegria™ Automaten. Hierbei wurden innerhalb eines Teststreifens jeweils die Probe und der teststreifeninterne Kalibrator mit 25OHD-Biotintracer und Vitamin D Freisetzungsreagenz gemischt, in die 250HD Antikörper beschichteten Kavitäten transferiert und nach 30 minütiger Inkubation gewaschen. Die Detektion des antikörpergebundenen Biotin-25OHD Tracers erfolgte mit Peroxidase-markiertem Steptavidin und einer Tetramethylbenzidin (TMB)-Farbreaktion durch Messung der optischen Dichte bei 650 nm.

Wie in **Abb. 3** dargestellt, wurde eine hohe Übereinstimmung zwischen den HPLC und LC-MS/MS definierten 25-OHD3/D2 Konzentrationen in Serenproben und dem direkten 25-OH Vitamin D3/D2 Bestimmungsverfahren im automatisierten Alegria™ System beobachtet.

### Beispiel 6: Bestimmung der Serumstabilität von 25-OH Vitamin D Derivaten

Zur vergleichenden Untersuchung der Serumstabilität von Biotin-25-OH Vitamin D Derivaten, wurden die Testverbindungen in Vitamin D-freier Serummatrix (Seracon, Seracare Inc.) aufgenommen. In einem ersten Ansatz (VD1) wurde das am C3-Atom in der nicht-natürlichen (R)-Konfiguration vorliegende 25-OH Vitamin D Derivat 3α-[α-Biotinamido-ω-dodeca(ethylenglycol)-amido]-25-hydroxy-deoxyvitamin D₃ gemäß Beispiel 1 auf eine Konzentration von 3 ng/ml in Vitamin D-freier Serummatrix eingestellt. In einem zweiten Ansatz (VD2) wurde das am C3-Atom in der natürlichen (S)-Konfiguration vorliegende 25-OH Vitamin D Derivat 3β-3'[α-Biotinamido-ω-dodeca(ethylenglycol)-amido]amidopropylether-25-hydroxy-vitamin D₃ gemäß US20080317764 auf eine Konzentration von 3 ng/ml in Vitamin D-freier Serummatrix eingestellt.

Aliquots von jeweils 1 ml der Ansätze VD1 und VD2 wurden bei -20 °C gelagert bzw. für 24 Stunden bei 37 °C inkubiert. Die Proben wurden anschließend für 1 Stunde bei Raumtemperatur (22 °C) äquilibriert. Je 500 µl der bei -20 °C gelagerten Referenzproben und der für 24 h bei 37 °C inkubierten Proben wurden mit je 500 µl Freisetzungsreagenz gemäß Beispiel 2 versetzt und gemischt. 100 µl der mit Vitamin D Freisetzungsreagenz verdünnten Proben wurden in 250HD-Antikörper beschichtete Kavitäten gemäß Beispiel 3 transferiert und 30 min bei Raumtemperatur inkubiert, so dass die in den Proben vorliegenden Biotin-25-OH Vitamin D Derivate an dem 250HD Antikörper binden.

Anschließend wurden die Probenansätze entfernt und die Kavitäten wurden 3 mal mit je 200 µl Waschpuffer gespült. Die Detektion der Antikörpergebundenen Biotin-25-OH Vitamin D Derivate erfolgte mit Peroxidase-markiertem Steptavidin und einer Tetramethylbenzidin (TMB)-Farbreaktion. Nach Abstoppen der Substratreaktion durch Zusatz von 100 µl 50 mM Phosphorsäure erfolgt die Messung der optischen Dichte bei 450 nm.

Hierbei zeigte sich, wie in **Abb. 4** dargestellt, mit dem 3α-[α-Biotinamido-ω-dodeca(ethylenglycol)-amido]-25-hydroxy-deoxyvitamin D₃ Derivat in Ansatz VD1 nach 24-stündiger Inkubation bei 37 °C gegenüber der bei -20 °C gelagerten Referenzprobe eine Wiederfindung von 95,2 %. Das in Ansatz VD2 vorliegende 3β-3'[α-Biotinamido-ω-dodeca(ethylenglycol)-amido] amidopropylether-25-hydroxy Vitamin D₃ Derivat weist eine deutlich geringere Serumstabilität auf. Hier wurde nach 24-stündiger Inkubation bei 37°C gegenüber der bei -20 °C gelagerten Referenzprobe nur eine Wiederfindung von 71,5 % nachgewiesen.

## Patentansprüche

1. Vitamin D-Verbindung mit einer Struktur nach Formel (I), 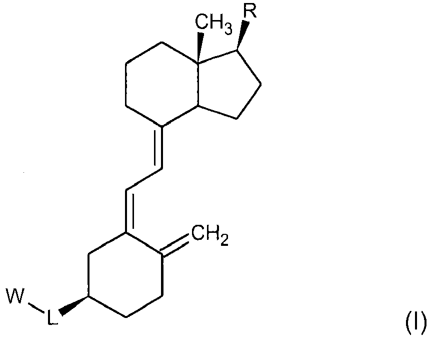 wobei
R ein C₂ - C₁₀ Alkyl- oder Alkenylrest ist, der gegebenenfalls durch eine Hydroxygruppe substituiert ist,
L ein Linker ist, und
W eine Markierungsgruppe ist, die keine Vitamin D-Verbindung ist.

2. Verbindung nach Anspruch 1, **dadurch gekennzeichnet, dass** der Linker eine Kettenlänge von 6 bis 110 Atomen, ausgewählt aus C-Atomen und gegebenenfalls Heteroatomen wie etwa N, O und/oder S aufweist, wobei die Kettenlänge bevorzugt 8 bis 80, 12 bis 60 oder 30 bis 50 Atome lang ist, und wobei L bevorzugt durch eine Struktur nach Formel (II) dargestellt ist: 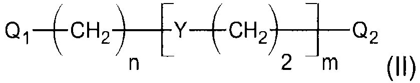 wobei
Q2: -C(O)NR¹-●, -C(O)O-●, -C(S)NR¹-●, -C(S)O-●, -C(O)S-●, -C(S)S-●, -O-●, -S-●, -NR¹-●, -CH₂O-●, -CH₂S-● oder -CH₂NR¹-● ist,
Q1: -C(O)-●, -NR²C(O)-●, -NR²C(S)-●, -OC(O)-●, -C(O)NR²-●, -C(S)NR²-●, -C(S)-●, -OC(S)-●, -SC(O)-●, -SC(S)-●, -O-●, -S-● oder **-**NR²**-**● ist,
Y: -O- oder -CH₂- ist,
R¹ und R² unabhängig voneinander H oder C₁- C₆ Alkyl sind, und
m 1 - 30 ist, und
n 1 - 12 ist,
wobei Q1 mit der Gruppe W und Q2 mit dem Grundkörper der Vitamin D-Verbindung nach Formel (I) verbunden ist und (●) jeweils die Verknüpfungstelle von Q2 mit dem Grundkörper der Vitamin D-Verbindung bzw. von Q1 mit der Gruppe W angibt.

3. Verbindung nach einem der Ansprüche 1 bis 2, **dadurch gekennzeichnet, dass** W Biotinyl, eine Fluoreszenzgruppe oder eine Chemolumineszenzgruppe ist, und/oder dass Y -O- ist.

4. Verbindung nach einem der Ansprüche 1 bis 3, **dadurch gekennzeichnet, dass**
Q2: -C(O)NR¹-● ist,
Q1: -C(O)-●, -NR²C(O)-●, -OC(O)-●, -C(O)NR²-●, -O-●, -S-● oder -NR²-● ist,
Y: -O- ist,
R¹ und R² unabhängig voneinander H oder C₁ - C₆ Alkyl und insbesondere H sind,
m 1 - 30 ist, und
n 1 - 12 ist.

5. Verbindung nach einem der Ansprüche 1 bis 4, **dadurch gekennzeichnet, dass** R eine Vitamin D-Seitenkette, mit einer Hydroxygruppe nach Formel (III) 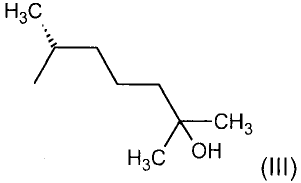 oder nach Formel (IV) 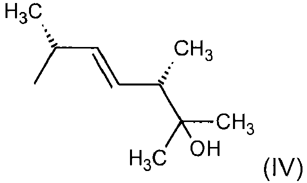 darstellt.

6. Verbindung nach einem der Ansprüche 1 bis 5 mit einer Struktur nach Formel (Ib), 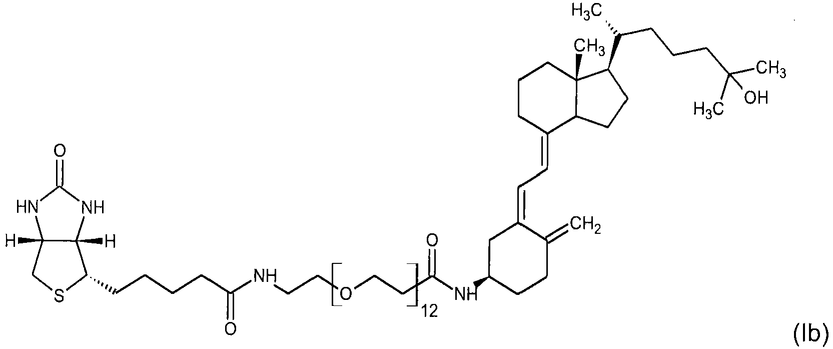 oder Formel (Ic),

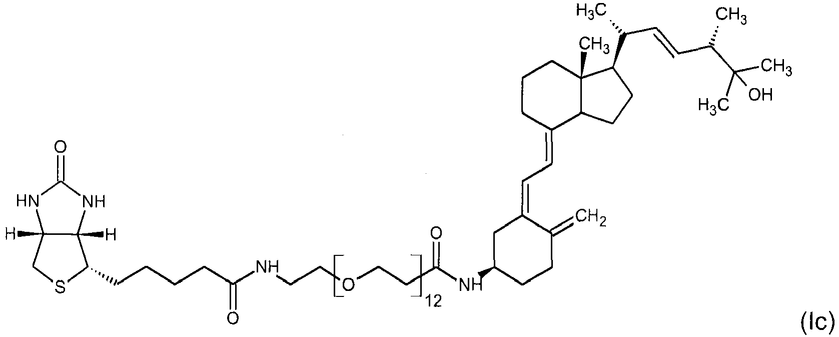

7. Verfahren zur Herstellung einer Verbindung nach einem der Ansprüche 1 bis 6, umfassend die Schritte:
(a) Durchführen einer nukleophilen Substitution an der Hydroxygruppe des C-3 Atoms einer Verbindung mit einer Struktur nach Formel (V) 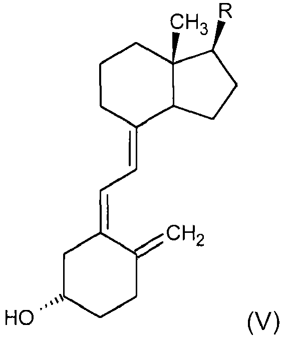 wobei R wie in einem der Ansprüche 1 bis 7 definiert ist,
wobei die Hydroxygruppe unter Inversion der Konfiguration am C-3 Stereozentrum durch eine Gruppe X ersetzt wird, um eine Verbindung mit einer Struktur nach Formel (VI) zu erhalten, 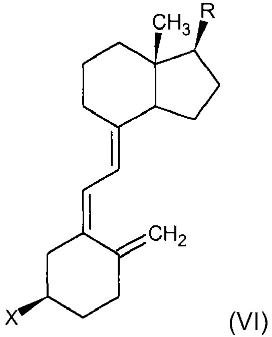 wobei R wie in einem der Ansprüche 1 bis 7 definiert ist und X eine gegebenenfalls geschützte Hydroxygruppe, Thiolgruppe oder primäre oder sekundäre Aminogruppe ist, und
(b) Kuppeln einer Markierungsgruppe W, die keine Vitamin D-Verbindung ist, über einen Linker an die Gruppe X der Verbindung mit einer Struktur nach Formel (VI), um eine Verbindung mit einer Struktur nach Formel (I) zu erhalten.

8. In-vitro-Verfahren zur quantitativen Bestimmung von Vitamin D in einer Probe, unter Verwendung einer Verbindung nach einem der Ansprüche 1 bis 6 als Tracer, wobei die zu untersuchende Probe bevorzugt eine biologische Flüssigkeit ist, die besonders bevorzugt ausgewählt wird aus Blut, Serum, Plasma oder Milch.

9. In-vitro-Verfahren nach Anspruch 8, umfassend die Schritte:
a) Inkontaktbringen einer Vitamin D enthaltenden Probe mit dem Tracer,
b) Quantitatives Bestimmen des Tracers unter Bedingungen, bei denen die detektierte Menge des Tracers einen Rückschluss auf die Gesamtkonzentration an Vitamin D in der Probe ermöglicht, wobei die Bestimmung des Tracers bevorzugt durch ein immunologisches Verfahren, besonders bevorzugt durch ein kompetitives immunologisches Verfahren, erfolgt.

10. Verfahren nach einem der Ansprüche 8 bis 9, **dadurch gekennzeichnet, dass** zur Bestimmung des Tracers ein Antikörper verwendet wird, welcher spezifisch die Seitenkette R erkennt und bevorzugt spezifisch eine Vitamin D3 oder Vitamin D2 Seitenkette, die an C25 eine Hydroxygruppe aufweist, erkennt.

11. Verfahren nach einem der Ansprüche 8 bis 10, **dadurch gekennzeichnet, dass** der Tracer der Probe in einer Menge zugesetzt wird, um eine Endkonzentration von 0,2 bis 100 ng/ml dieser Verbindung einzustellen.

12. Verfahren nach einem der Ansprüche 8 bis 11, **dadurch gekennzeichnet, dass** das zu bestimmende Vitamin D am C-25 eine Hydroxygruppe aufweist und ausgewählt wird aus der Gruppe umfassend 25-Hydroxyvitamin D3, 25-Hydroxyvitamin D2, 1,25-Dihydroxyvitamin D3, 1,25-Dihydroxyvitamin D2 und Mischungen davon, wobei das Vitamin D bevorzugt 25-Hydroxyvitamin D3 und/oder 25-Hydroxyvitamin D2 ist.

13. Reagenz zur Bestimmung von Vitamin D, umfassend eine Verbindung nach einem der Ansprüche 1 bis 6, gegebenenfalls weiterhin umfassend ein Reagenz zur Freisetzung von gebundenem Vitamin D und gegebenenfalls Verdünnungsmittel, Täger, Lösemittel, Adjuvantien, Stabilisatoren, Konservierungsmittel und/oder Dispergiermittel.

14. Verwendung einer Verbindung nach einem der Ansprüche 1 bis 6 oder eines Reagenz nach Anspruch 13 zur Bestimmung von Vitamin D, insbesondere in einem Verfahren nach einem der Ansprüche 8 bis 12.

15. Verwendung einer Verbindung mit einer Struktur nach Formel (VI): 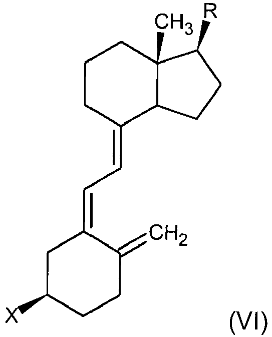
wobei R ein C₂ - C₁₀ Alkyl- oder Alkenylrest ist, der gegebenenfalls durch Hydroxygruppe substituiert ist, und
X NHR³, OH oder SH ist,
wobei R³ H oder C₁ - C₆ Alkyl ist,
zur Herstellung einer Verbindung mit einer Struktur nach Formel (I), Formel (Ib) oder Formel (Ic).

## Claims

1. Vitamin D compound having a structure according to formula (I), 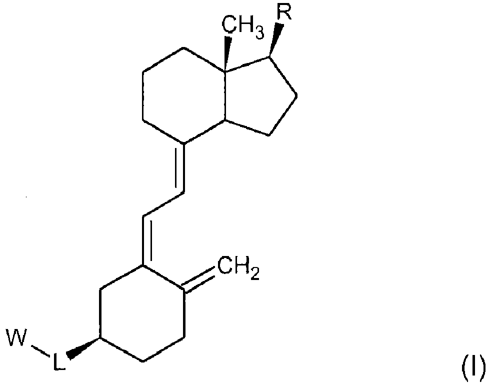 wherein
R is a C₂-C₁₀ alkyl functional group or alkenyl functional group, which is optionally substituted by a hydroxy group,
L is a linker, and
W is a labelling group that is not a vitamin D compound.

2. Compound according to claim 1, **characterised in that** the linker has a chain length of from 6 to 110 atoms, selected from C atoms and optionally heteroatoms such as N, O and/or S, the chain length preferably being 8 to 80, 12 to 60 or 30 to 50 atoms, and L preferably being represented by a structure according to formula (II): 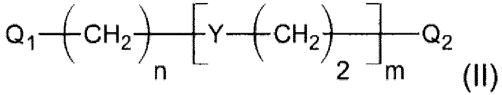 where
Q2 is: -C(O)NR¹-●, -C(O)O-●, -C(S)NR¹-●, -C(S)O-●, -C(O)S-●, -C(S)S-●, -O-●, -S-●, -NR¹-●, -CH₂O-●, -CH₂S-● or-CH₂NR¹-●,
Q1 is: -C(O)-●, -NR²C(O)-●, -NR²C(S)-●, -OC(O)-●, -C(O)NR²-●, -C(S)NR²-●, -C(S)-●, -OC(S)-●, -SC(O)-●, -SC(S)-●, -O-●, -S-● or-NR²-●,
Y is: -O- or -CH₂-,
R¹ and R², independently of one another, are H or C₁-C₆ alkyl, and
m is 1-30, and
n is 1-12,
Q1 being bonded to the group W and Q2 being bonded to the parent substance of the vitamin D compound according to formula (I), and in each case (●) representing the binding site of Q2 to the parent substance of the vitamin D compound or of Q1 to the group W.

3. Compound according to either claim 1 or claim 2, **characterised in that** W is biotinyl, a fluorescent group or a chemiluminescent group, and/or **in that** Y is -O-.

4. Compound according to any of claims 1 to 3, **characterised in that**
Q2 is: -C(O)NR¹-●,
Q1 is: -C(O)-●, -NR²C(O)-●, -OC(O)-●, -C(O)NR²-●, -O-●, -S-● or-NR²-●,
Y is: -O-,
R¹ and R², independently of one another, are H or C₁-C₆ alkyl and in particular H,
m is 1-30, and
n is 1-12.

5. Compound according to any of claims 1 to 4, **characterised in that** R represents a vitamin D side chain, having a hydroxy group according to formula (III) 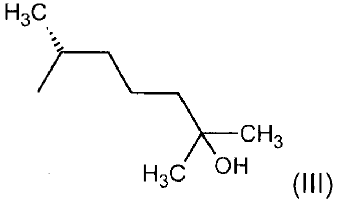 or according to formula (IV)

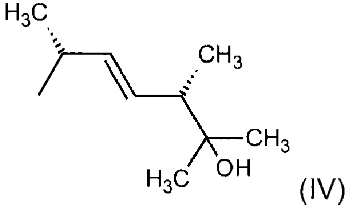

6. Compound according to any of claims 1 to 5 having a structure according to formula (Ib), 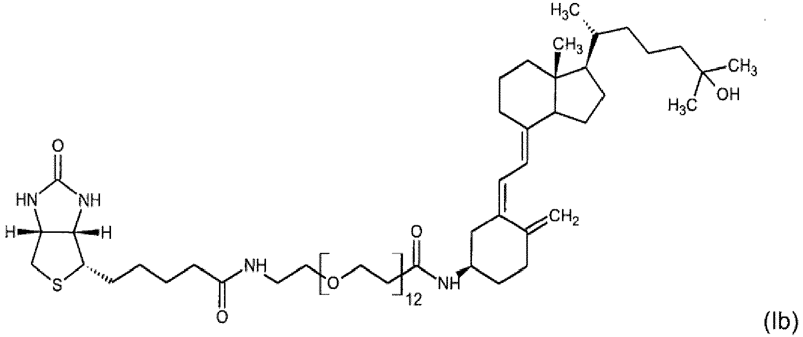 or formula (Ic)

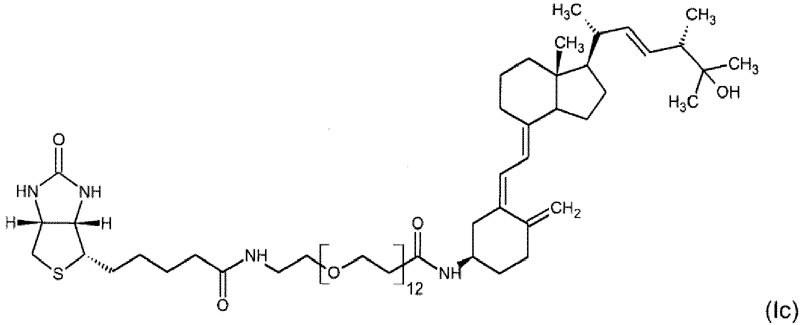

7. Method for producing a compound according to one of claims 1 to 6, comprising the steps of:
(a) carrying out a nucleophilic substitution at the hydroxy group of the C3 atom of a compound having a structure according to formula (V), 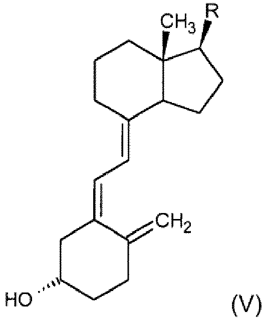 wherein R is defined as in any of claims 1 to 7,
wherein the hydroxy group is replaced by a group X with inversion of the configuration at the C3 stereocentre, in order to obtain a compound having a structure according to formula (VI), 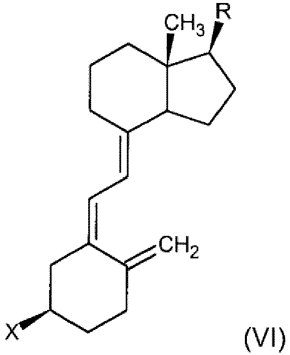 wherein R is defined as in any of claims 1 to 7 and X is an optionally protected hydroxy group, thiol group, or primary or secondary amino group, and
(b) coupling a labelling group W which is not a vitamin D compound to the group X of the compound having a structure according to formula (VI) via a linker, in order to obtain a compound having a structure according to formula (I).

8. In-vitro method for quantitatively determining vitamin D in a sample, using a compound according to any of claims 1 to 6 as a tracer, wherein the sample to be tested is preferably a biological fluid which is particularly preferably selected from blood, serum, plasma or milk.

9. In-vitro method according to claim 8, comprising the steps of:
a) bringing a sample containing vitamin D into contact with the tracer,
b) quantitatively determining the tracer under conditions in which the detected quantity of tracer allows a conclusion to be drawn as to the overall concentration of vitamin D in the sample, wherein the tracer is preferably determined by an immunological method, particularly preferably by a competitive immunological method.

10. Method according to either claim 8 or claim 9, **characterised in that** an antibody which specifically recognises the side chain R and preferably specifically recognises a vitamin D3 or vitamin D2 side chain having a hydroxy group at C25 is used to determine the tracer.

11. Method according to any of claims 8 to 10, **characterised in that** the tracer is added to the sample in a quantity so as to establish a final concentration of from 0.2 to 100 ng/ml of said compound.

12. Method according to any of claims 8 to 11, **characterised in that** the vitamin D to be determined comprises a hydroxy group at C25 and is selected from the group comprising 25-hydroxy vitamin D3, 25-hydroxy vitamin D2, 1,25-dihydroxy vitamin D3, 1,25-dihydroxy vitamin D2 and mixtures thereof, the vitamin D preferably being 25-hydroxy vitamin D3 and/or 25-hydroxy vitamin D2.

13. Reagent for determining vitamin D, comprising a compound according to any of claims 1 to 6, optionally further comprising a reagent for releasing bound vitamin D and optionally diluting agents, carriers, solvents, adjuvants, stabilisers, preservatives and/or dispersants.

14. Use of a compound according to any of claims 1 to 6 or of a reagent according to claim 13 for determining vitamin D, in particular in a method according to any of claims 8 to 12.

15. Use of a compound having a structure according to formula (VI): 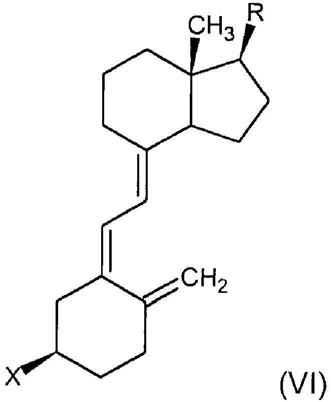
wherein R is a C₂-C₁₀ alkyl functional group or alkenyl functional group, which is optionally substituted by a hydroxy group, and
X is NHR³, OH or SH,
wherein R³ is H or a C₁-C₆ alkyl,
for producing a compound having a structure according to formula (I), formula (Ib) or formula (Ic).

## Revendications

1. Composé de la vitamine D ayant une structure selon la formule (I) 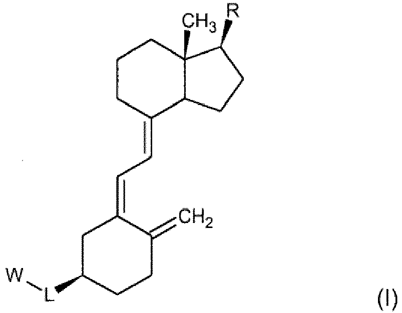 où
R est un radical C₂-C₁₀ alkyle ou alcényle, qui est éventuellement substitué par un groupe hydroxyle,
L est un lieur, et
W est un groupe de marquage, qui n'est pas un composé de la vitamine D.

2. Composé selon la revendication 1, **caractérisé en ce que** le lieur présente une longueur de chaîne de 6 à 110 atomes, choisis parmi les atomes C et éventuellement les hétéroatomes comme par exemple N, O et/ou S, où la longueur de chaîne est de préférence longue de 8 à 80, 12 à 60 ou 30 à 50 atomes, et où L est représenté de préférence par une structure selon la formule (II): 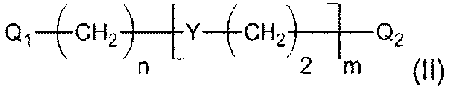 où
Q2 est: -C(O)NR¹-●, -C(O)O-●, -C(S)NR¹-●, -C(S)O-●, -C(O)S-●, -C(S)S-●, -O-●, -S-●, -NR¹-●, -CH₂O-●, -CH₂S-● ou -CH₂NR¹-●,
Q1 est: -C(O)-●, -NR²C(O)-●, -NR²C(S)-●, -OC(O)-●, -C(O)NR²-●, -C(S)NR²-●, -C(S)-●, -OC(S)-●, -SC(O)-●, -SC(S)-●, -O-●, -S-● ou -NR²-●,
Y est: -O- ou -CH₂-,
R¹ et R² sont indépendamment l'un de l'autre H ou C₁-C₆ alkyle, et
m est 1 - 30, et
n est 1 - 12,
où Q1 est lié au groupe W et Q2 est lié au squelette fondamental du composé de la vitamine D selon la formule (I) et (●) indique dans chaque cas le site de liaison de Q2 au squelette fondamental du composé de la vitamine D resp. de Q1 au groupe W.

3. Composé selon l'une des revendications 1 à 2, **caractérisé en ce que** W est biotinyle, un groupe fluorescent ou un groupe chimiluminescent, et/ou **en ce que** Y est -O-.

4. Composé selon l'une des revendications 1 à 3, **caractérisé en ce que**
Q2 est: -C(O)NR¹-●,
Q1 est: -C(O)-●, -NR²C(O)-●, -OC(O)-●, -C(O)NR²-●, -O-●, -S-● ou -NR²-●,
Y est: -O-,
R¹ et R² sont indépendamment l'un de l'autre H ou C₁-C₆ alkyle et en particulier H,
m est 1 - 30, et
n est 1 - 12.

5. Composé selon l'une des revendications 1 à 4, **caractérisé en ce que** R représente une chaîne latérale de la vitamine D ayant un groupe hydroxyle selon la formule (III) 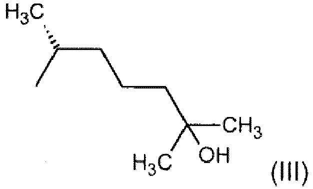 ou selon la formule (IV)

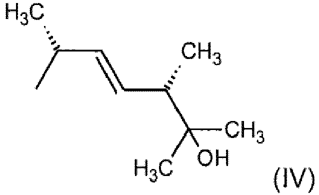

6. Composé selon l'une des revendications 1 à 5 ayant une structure selon la formule (Ib) 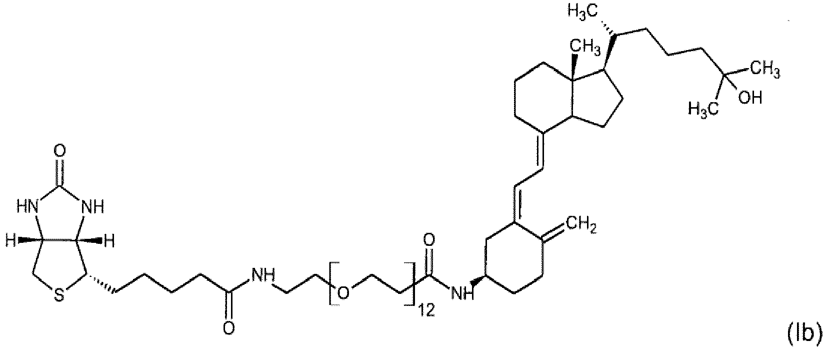 ou la formule (Ic)

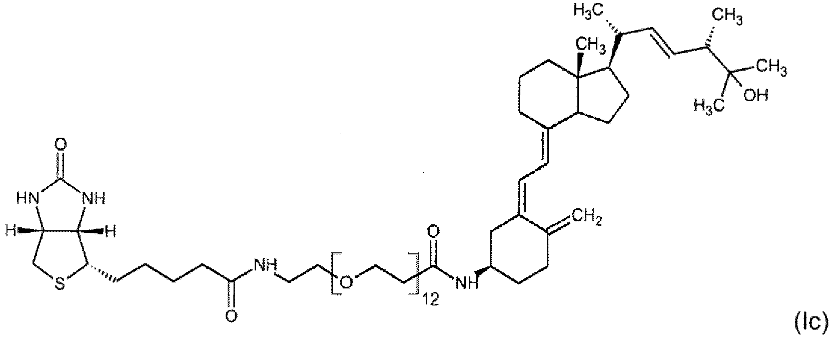

7. Procédé pour la production d'un composé selon l'une des revendications 1 à 6, comprenant les étapes:
(a) conduite d'une substitution nucléophile au niveau du groupe hydroxyle de l'atome C-3 d'un composé ayant une structure selon la formule (V) 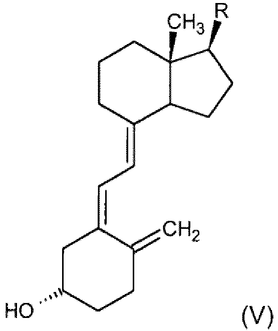 où R est défini comme dans l'une des revendications 1 à 7,
où le groupe hydroxyle est remplacé par un groupe X avec inversion de la configuration au niveau du stéréocentre C-3, pour obtenir un composé ayant une structure selon la formule (VI) 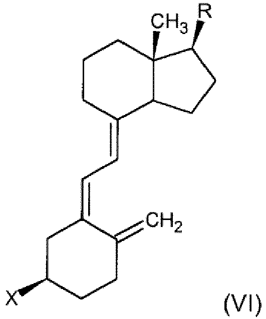 où R est défini comme dans l'une des revendications 1 à 7 et X est un groupe hydroxyle, un groupe thiol ou un groupe amino primaire ou secondaire éventuellement protégé, et
(b) couplage d'un groupe de marquage W, qui n'est pas un composé de la vitamine D, par le biais d'un lieur au groupe X du composé ayant une structure selon la formule (VI), pour obtenir un composé ayant une structure selon la formule (I).

8. Procédé in vitro pour la détermination quantitative de la vitamine D dans un échantillon, au moyen d'un composé selon l'une des revendications 1 à 6 comme traceur, où l'échantillon destiné à être étudié est de préférence un liquide biologique, qui est choisi de manière particulièrement préférée parmi le sang, le sérum, la plasma ou le lait.

9. Procédé in vitro selon la revendication 8, comprenant les étapes:
a) mise en contact d'un échantillon contenant de la vitamine D avec le traceur,
b) détermination quantitative du traceur dans des conditions dans lesquelles la quantité détectée du traceur permet de déduire la concentration totale en vitamine D dans l'échantillon, où la détermination du traceur a lieu de préférence par un procédé immunologique, de manière particulièrement préférée par un procédé immunologique compétitif.

10. Procédé selon l'une des revendications 8 à 9, **caractérisé en ce que**, pour la détermination du traceur, un anticorps est utilisé, qui reconnaît spécifiquement la chaîne latérale R et de préférence qui reconnaît spécifiquement une chaîne latérale de la vitamine D3 ou de la vitamine D2 qui présente un groupe hydroxyle en C25.

11. Procédé selon l'une des revendications 8 à 10, **caractérisé en ce que** le traceur est ajouté à l'échantillon en une quantité pour établir une concentration finale de 0,2 à 100 ng/ml de ce composé.

12. Procédé selon l'une des revendications 8 à 11, **caractérisé en ce que** la vitamine D destinée à être déterminée présente en C-25 un groupe hydroxyle et est choisie dans le groupe comprenant la 25-hydroxyvitamine D3, la 25-hydroxyvitamine D2, la 1,25-dihydroxyvitamine D3, la 1,25-dihydroxyvitamine D2 et les mélanges de celles-ci, où la vitamine D est de préférence la 25-hydroxyvitamine D3 et/ou la 25-hydroxyvitamine D2.

13. Réactif pour la détermination de la vitamine D, comprenant un composé selon l'une des revendications 1 à 6, comprenant éventuellement en outre un réactif pour la libération de la vitamine D liée et éventuellement des diluants, des vecteurs, des solvants, des adjuvants, des stabilisants, de conservateurs et/ou des dispersants.

14. Utilisation d'un composé selon l'une des revendications 1 à 6 ou d'un réactif selon la revendication 13 pour la détermination de la vitamine D, en particulier dans un procédé selon l'une des revendications 8 à 12.

15. Utilisation d'un composé ayant une structure selon la formule (VI): 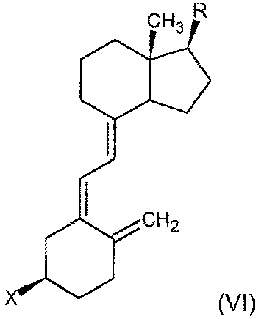
où R est un radical C₂-C₁₀ alkyle ou alcényle, qui est éventuellement substitué par un groupe hydroxyle, et
X est NHR³, OH ou SH,
où R³ est H ou C₁-C₆ alkyle,
pour la production d'un composé ayant une structure selon la formule (I), la formule (Ib) ou la formule (Ic).
